# EUROPEAN PATENT SPECIFICATION

(11) **EP 2 877 203 B1**
(45) Date of publication and mention of the grant of the patent: **17.10.2018**
(21) Application number: 13745370.0
(22) Date of filing: 22.07.2013
(51) Int. Cl.: A61K 38/48, A61K 31/12, A61K 31/353, A61K 31/4525, A61K 33/04, A61K 33/32, A61P 29/00

(54) **COMPOSITIONS FOR ORAL ADMINISTRATION HAVING A BENEFICIAL EFFECT ON TENDINOPATHIES AND LIGAMENT INJURIES**
ZUSAMMENSETZUNGEN ZUR ORALEN VERABREICHUNG MIT GÜNSTIGER WIRKUNG AUF TENDINOPATHIEN UND BÄNDERVERLETZUNGEN
COMPOSITIONS POUR ADMINISTRATION ORALE AYANT UN EFFET BÉNÉFIQUE SUR DES TENDINOPATHIES ET DES LÉSIONS DE LIGAMENT

(30) Priority: 25.07.2012 IT MI20121298
(43) Date of publication of application: 03.06.2015
(73) Proprietor: Sochim International SpA, 20010 Cornaredo (MI) (IT)
(72) Inventor: EIGENMANN, Carlo, I-20149 Milano (IT); PACCHETTI, Barbara, I-20154 Milano (IT)
(74) Representative: Villa, Livia
(86) International application number: PCT/EP2013/065396
(87) International publication number: WO 2014/016238

(56) References cited:
- WO-A2-2006/128032

## Description

### Summary of the invention

The present invention relates to compositions for oral administration having a beneficial effect on tendinopathies and ligament injuries, containing as active ingredients curcumin or a mixture of curcuminoids, catechin extract or epigallocatechin gallate, bromelain, and an agent selected from selenium and manganese, preferably in combination with coenzyme Q10.

### Technical background

Tendons are fibrous elastic bundles that connect the muscles to the bones, and assist bone mobility after a muscle contraction. They consist of a highly organised fibrillary matrix, the ingredients of which are mainly type I collagen, various other "minor" forms of collagen, proteoglycans and glycoproteins. The cell component mainly consists of tenocytes, specialist fibroblasts responsible for matrix secretion, assembly of collagen and its turnover in the tendon. The tendons are sheathed in peritendineum, a thin layer of connective tissue that penetrates into them, providing compactness and mutual adherence between the tendon fibres.

The main function of the tendon matrix is to transmit the load of tension to which the tendon is subjected, stabilise the joint and protect the muscle. The functionality of the tendon matrix is ensured by a finely regulated continuous process of degradation and remodelling, which presents some variability of turnover according to the site involved, and is mediated by the action of enzymes, such as matrix metalloproteinase (MMP) and other ADAM (A disintegrin and metalloproteinase) proteases, responsible for the degradation of collagen and proteoglycan in healthy volunteers and in patients suffering from tendinopathies.

It has been observed that the appearance of tendinopathies is associated with alterations in the matrix remodelling process. The main alterations at molecular level are characterised by increased expression of type III collagen, fibronectin, tenascin-C, aggrecan and biglycan. Although under normal conditions these alterations are the expression of tendon repair, they can also indicate an adaptive response to stresses caused by variations in mechanical load. It is believed that repeated, though minimal stresses trigger tendinopathies, although further studies are needed to determine whether the alteration in tenocyte activity is due to over- or under-stimulation.

Riley G. (Nature Clinical Practice Rheumatology (2008) Vol 4 N.2) has demonstrated that in tendinopathies, there is over-expression of metalloproteinases such as ADAM (A disintegrin and metalloproteinase)-12 and MMP (matrix metalloproteinase)-23 in the damaged tendon, and a concomitant variation in the expression of other molecules, such as type III collagen, fibronectin and some cytokines. Although the role of these enzymes in tendinopathies is not yet fully understood, it can be postulated that an imbalance in the homeostasis of the tendon matrix is a crucial factor in the mechanism of onset of tendinopathies.

The term "tendinopathy" refers generically to disorders affecting the tendons. The most common are tendinitis and tendinosis, a degenerative disorder of the tendon.

In particular, tendinitis or peritendinitis, an inflammation of the peritendineum sheath that surrounds the tendon, is connected with factors such as wear, microtraumas, continual microstresses, excessive fatigue, excess weight, and continuous use of medicaments (such as corticosteroids, ciprofloxacin and statins), which can cause the onset of an inflammatory process that may damage the tendons. Despite the tendency of the tissues to restore damaged parts, the original integrity and elasticity of the tendon are no longer restored, so that it becomes less responsive to continual motor stimuli.

Tendinopathies are one of the main causes of musculoskeletal pain, are conditions with a mainly degenerative nature, and frequently have a chronic course. These disorders are very widespread, and relatively difficult to treat.

Like tendons, ligaments are fibrous structures mainly consisting of collagen, but their function is to connect two bones . Ligaments are characterised by high tensile strength, but low elasticity. The ligaments perform their function by preventing alterations in the position of the structures to which they are connected when said structures are subjected to extreme movements, thus maintaining the integrity of the joint.

When the ligaments are subjected to excessive stresses they stretch, then gradually tear, until they completely rupture. Ligament injuries are classified, on the basis of their extent, as first-degree (microscopic lesions affecting a small part of the fibres), second-degree (lesions affecting at least half the fibres) and third-degree (complete rupture of the ligament in the central region or at the point of insertion into the bone).

Ligament injuries involve instability of the joint proportional to the extent of the lesion. Ligament injuries are often associated with haemorrhage in the joint space or pooling of synovial fluid, which causes swelling, bruising and pain.

Treatment at the acute stage consists of joint rest, application of ice and anti-inflammatory treatment. This is generally followed by rehabilitative physiotherapy. In particular cases, such as rupture of the anterior cruciate ligament of the knee, surgery is necessary.

Due to their good vascularisation, the ligaments have a reasonably good repair capacity.

However, recovery always takes a relatively long time: up to as much as 3 months. Anti-inflammatory treatment is therefore used in most cases.

The treatment of the above-mentioned conditions is usually based on the administration of non-steroidal anti-inflammatory drugs and physiotherapy; however, the efficacy of these treatment strategies is currently controversial. The associated anti-inflammatory treatment has limited benefits, as demonstrated by some clinical trials (Almekinders LC and Temple JD (1998) Etiology, diagnosis, and treatment of tendinitis: an analysis of the literature. Med Sci Sports Exerc 30: 1183-1190). Moreover, anti-inflammatories, while often effective in providing temporary pain relief, are responsible for a long series of secondary effects, which increase as the treatment proceeds.

Bromelain, an enzyme extracted from pineapple stems, is used in the acute treatment of pain and inflammation of the soft tissues following mechanical lesions. Aiyegbusi A.I. et al. (Phytother. Res. 25: 49-52 (2011) have demonstrated that bromelain has a favourable effect at the initial stages of healing of Achilles tendon injuries, stimulating tenocyte proliferation and acting on the malondialdehyde levels. The same authors compared the effect of bromelain and fresh pineapple juice on tenocyte proliferation and malondialdehyde levels in the initial stages of healing of Achilles tendon injuries, and found that bromelain had a greater effect (Aiyegbusi AI, et al (J Med Food. 2011 Apr;14(4):348-52. Epub 2011 Jan 23).

Corpsa, A.N., et al. (Matrix Biology 23 (2004) 163-169) described the inhibiting effects of epigallocatechin gallate (EGCG) on the expression of collagenase, matrix metalloproteinase (MMP) and stromelysin (MMP-3) induced by interleukin-lb in human tendon fibroblasts, suggesting that breakdown of the extracellular matrix may constitute a potential therapeutic target for the action of green tea polyphenols.

Buhrmann c. et al (The Journal of Biological Chemistry vol. 286, NO. 32, pp. 28556-28566, August 12, 2011) have suggested a potential role for curcumin in the treatment of tendon inflammations due to the down-regulation action on the gene products involved in tenocyte apoptosis, matrix breakdown and inflammation.

### Description of the invention

It has now been found that compositions containing curcumin or a mixture of curcuminoids, bromelain, catechin extract or epigallocatechin gallate, piperine, and an agent selected from selenium and manganese, have a favourable effect on tendinopathies and ligament injuries.

The present invention therefore relates to compositions containing:
a) curcumin or a mixture of curcuminoids,
b) bromelain,
c) catechin extract or epigallocatechin gallate,
d) piperine, and
e) an agent selected from selenium and manganese
and their use in the treatment of tendinopathies and ligament injuries.

More particularly, the present invention relates to nutraceutical, dietary and nutritional compositions having a beneficial effect on tendon disorders, an anti-inflammatory action, a homeostasis promotion and maintenance action on the tendon matrix, and an antioxidant and protective action on the tendon structures, for the treatment of tendinopathies and ligament injuries.

It has now surprisingly been found that the compositions of the invention exhibit a greater effect than that obtained from the sum of the effects deriving from separate administration of the individual ingredients. It is postulated that said greater effect originates from synergy between the various ingredients of the composition, but the invention is obviously not tied to the action mechanism underlying the effect described. Due to said synergic effect, the compositions of the invention are also active at relatively low doses, which meet the specifications for food supplements.

The compositions could be used directly in the form of tablets, capsules, soft gel capsules, orodispersible granulates, powders for extempore use and liquid preparations, or to prepare food supplements.

According to a preferred aspect, the compositions of the invention will contain turmeric extract with a minimum content of 95% curcumin or total curcuminoids, in quantities ranging from about 250 mg to about 3000 mg. A controlled-release curcumin matrix can also be used. Said matrix is available on the market, and presents a curcumin content of about 25%. However, various other sources of curcumin with different standardization could obviously be used.

According to a preferred aspect, the compositions of the invention will contain bromelain or a pineapple stem extract containing it, in quantities such as to provide from about 100 GDU (Gelatin Digestive Units) or equivalent to about 1000 GDU or equivalent.

According to a preferred aspect, the compositions of the invention will contain catechin extract or epigallocatechin gallate from at least 90% green tea extract, in quantities ranging from about 25 mg to about 300 mg

According to a preferred aspect, the compositions of the invention will contain piperine from at least 95% pepper extract, in quantities ranging from about 0.5 mg to about 5 mg.

According to a preferred aspect, the compositions of the invention will contain selenium in quantities ranging from about 8.25 mcg to about 83 mcg, or manganese in quantities ranging from about 0.3 mg to about 10 mg.

According to a preferred aspect, the compositions of the invention will also contain coenzyme Q10, in quantities ranging from about 5 mg to about 200 mg.

The compositions of the invention could also contain additional ingredients with a complementary or otherwise favourable action, such as, but not limited to, collagen in its various forms (native, hydrolysed, type I, II or III), methylsulphonylmethane, aminoacids and derivatives thereof (such as lysine and ornithine), alpha-ketoglutaric acid and salts thereof, proanthocyanidins and polyphenols, lipoic acid, superoxide dismutase, vitamins C, E and group B, glucosamine, and glycosaminoglycans such as hyaluronic acid or chondroitin sulphate.

The compositions of the invention could be formulated suitably for oral administration, and prepared according to conventional methods well known in pharmaceutical technology, using excipients, diluents, fillers and other carriers acceptable for their final use.

The following is an example of a formulation according to the invention.

| **COMPOSITION** | **PER TABLET** | **% RDA* PER TABLET** |
|---|---|---|
| Turmeric extract (95% curcumin) | 500 mg | - |
| Natural bromelain from pineapple stem extract | 300 GDU or 163 mg | - |
| Epigallocatechin gallate from 90% green tea extract | 25 mg | - |
| Piperine from 95% pepper extract | 2.5 mg | - |
| Selenium | 41.5 mcg | 75% |
| Manganese | 2 mg | 100% |
| Coenzyme Q10 | 10 mg | - |

| | | |
|---|---|---|
| *RDA = Recommended Dietary Allowance | | |

### BIBLIOGRAPHY

Riley G (2008) Review. Tendinopathies - from basic science to treatment. Nature Clinical Practice Rheumatology Vol.4 N.2 (www.nature.com/clinicalpractice/rheum.
Almekinders LC and Temple JD (1998) Etiology, diagnosis, and treatment of tendinitis: an analysis of the literature. Med Sci Sports Exerc 30: 1183-1190.
Buhrmann C et al, Curcumin modulates Nuclear Factor kB (NF-kB)-mediated inflammation in human tenocytes in vitro, J Biol Chem, 2011.
Aiyegbusi AI et al, Bromelain in the early phase of healing in acute crush Achilles tendon injury, Phytother Res, 2011.
Aiyegbusi AI et al, A comparative study of the effects of bromelain and fresh pineapple juice on the early phase of healing in acute crush Achilles tendon injury, J Med Food, 2011.
Corps AN et al, Inhibition of interleukin-1b-stimulated collagenase and stromelysin expression in human tendon fibroblasts by epigallocatechin gallate ester, Matrice Biology, 2004.

## Claims

1. Compositions comprising:
a) curcumin or a curcuminoid mixture,
b) bromelain or an extract of pineapple stem,
c) catechin extract or epigallocatechin gallate,
d) piperine, and
e) an agent selected from selenium and manganese.

2. Compositions according to claim 1, comprising the active ingredients within the following ranges by weight per unit dose:
a) curcuma extract containing minimum 95% curcumin or total curcuminoids, in amounts ranging from 250 mg to 3000 mg,
b) bromelain: from 100 Gelatin Digestive Units (or equivalents) to 1000 Gelatin Digestive Units (or equivalents),
c) catechin extract or epigallocatechin gallate: from 25 mg to 300 mg,
d) piperine: from 0.5 mg to 5 mg, and
e) selenium in amounts ranging from 8.25 µg to 83 µg, or manganese in amounts ranging from 0.3 mg to 10 mg.

3. Compositions according to claims 1 and 2, further comprising coenzyme Q10.

4. Compositions according to claim 3, comprising coenzyme Q10 in amounts ranging from 5 mg to 200 mg.

5. Compositions according to the above claims, for use in the treatment of tendinopathies and ligament lesions.

## Patentansprüche

1. Zusammensetzungen, die Folgendes umfassen:
a) Curcumin oder eine Curcuminoidmischung,
b) Bromelain oder einen Extrakt vom Ananasstamm,
c) Catechin-Extrakt oder Epigallocatechin-Gallat,
d) Piperin, und
e) ein Mittel ausgewählt aus Selenium und Mangan.

2. Zusammensetzungen nach Anspruch 1, umfassend die aktiven Inhaltsstoffe innerhalb der folgenden Bereiche nach Gewicht pro Dosierungseinheit:
a) Curcuma-Extrakt, der mindestens 95 % Curcumin oder Gesamtcurcuminoide enthält, in Mengen im Bereich von 250 mg bis 3000 mg,
b) Bromelain: 100 Gelatine-Verdauungseinheiten (oder Äquivalenten) bis 1000 Gelatine-Verdauungseinheiten (oder Äquivalenten),
c) Catechinextrakt oder Epigallocatechin-Gallat: 25 mg bis 300 mg,
d) Piperin: 0,5 mg bis 5 mg, und
e) Selenium in Mengen im Bereich von 8,25 µg bis 83 µg, oder Mangan in Mengen im Bereich von 0,3 mg bis 10 mg.

3. Zusammensetzung nach den Ansprüchen 1 und 2, ferner umfassend Koenzym Q10.

4. Zusammensetzungen nach Anspruch 3, enthaltend Koenzym Q10 in Mengen im Bereich von 5 mg bis 200 mg.

5. Zusammensetzungen nach einem der vorhergehenden Ansprüche zur Verwendung in der Behandlung von Tendinopathien und Bänderläsionen.

## Revendications

1. Compositions comprenant :
a) de la curcumine ou un mélange de curcuminoïdes,
b) de la bromélaïne ou un extrait de tige d'ananas,
c) un extrait de catéchine ou du gallate d'épigallocatéchine,
d) de la pipérine et
e) un agent choisi parmi le sélénium et le manganèse.

2. Compositions selon la revendication 1, comprenant les ingrédients actifs dans les plages suivantes par poids par dose unitaire :
a) extrait de curcuma contenant au moins 95 % de curcumine ou de curcuminoïdes totaux, en des quantités variant de 250 mg à 3000 mg,
b) bromélaïne : de 100 unités de digestion de gélatine (ou équivalents) à 1000 unités de digestion de gélatine (ou équivalents),
c) extrait de catéchine ou gallate d'épigallocatéchine : de 25 mg à 300 mg,
d) pipérine : de 0,5 mg à 5 mg, et
e) sélénium en quantités variant de 8,25 µg à 83 µg ou manganèse en quantités variant de 0,3 mg à 10 mg.

3. Compositions selon les revendications 1 et 2, comprenant en outre la coenzyme Q10.

4. Compositions selon la revendication 3, comprenant la coenzyme Q10 en quantités variant de 5 mg à 200 mg.

5. Compositions selon les revendications ci-dessus, destinées à être utilisées dans le traitement de tendinopathies et de lésions de ligament.
